# FASCICULE DE BREVET EUROPEEN

(11) **EP 1 380 284 B1**
(45) Date de publication et mention de la délivrance du brevet: **29.06.2005**
(21) Numéro de dépôt: 03291647.0
(22) Date de dépôt: 03.07.2003
(51) Int. Cl.: A61K 7/075, A61K 7/06

(54) **Compositions cosmétiques détergentes contenant un tensioactif anionique, un tensioactif amphotère ou non ionique et un polysaccharide et utilisation**
Kosmetische Reinigungszusammensetzungen, die ein anionisches, ein amphoterisches oder nichtionischesTensid und ein Polysaccharid enthalten, und deren Verwendung
Cosmetic detergent compositions containing an anionic surfactant, an amphoteric or nonionic surfactant and a polysaccharide, and use thereof

(30) Priorité: 08.07.2002 FR 0208553
(43) Date de publication de la demande: 14.01.2004
(73) Titulaire: L'Oreal, 75014 Paris (FR)
(72) Inventeur: Lazzeri, Pascale, 92300 Levallois Perret (FR); Apvrille, Alice, 92300 Levallois, Perret (FR)
(74) Mandataire: Le Blainvaux Bellegarde, Françoise

(56) Documents cités:
- FR-A- 2 795 951
- FR-A- 2 795 953

## Description

La présente invention concerne de nouvelles compositions cosmétiques à propriétés améliorées destinées simultanément au nettoyage et au conditionnement des cheveux, et comprenant, dans un support cosmétiquement acceptable, un tensioactif anionique, un tensioactif amphotère, non ionique et/ou cationique et au moins un polysaccharide choisi parmi les hydrolysats d'amidon et les fructanes non ioniques ou anioniques.
L'invention conceme aussi l'utilisation desdites compositions dans l'application cosmétique susmentionnée.

Pour le nettoyage et/ou le lavage des cheveux, l'utilisation de compositions capillaires détergentes (ou shampooings) à base essentiellement d'agents tensioactifs classiques notamment anioniques, non-ioniques et/ou amphotères, mais plus particulièrement anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.
Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés de silicone, qui confèrent en effet aux cheveux lavés, secs ou mouillés, une facilité de démêlage et une douceur nettement accrues par rapport à ce qui peut être obtenu avec les compositions nettoyantes correspondantes qui en sont exemptes.

Cependant, les avantages cosmétiques mentionnés ci-avant s'accompagnent malheureusement également, sur cheveux séchés, de certains effets cosmétiques jugés indésirables, à savoir notamment un alourdissement de la coiffure, un manque de lissage.
En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

En résumé, il s'avère que les compositions cosmétiques actuelles contenant des agents conditionneurs, ne donnent pas complètement satisfaction.

Sur des cheveux sensibilisés, afin d'obtenir les effets cosmétiques des silicones sur toute la longueur de la fibre capillaire, on utilise de préférence des associations de silicones et de polymères cationiques.

Toutefois, et malgré les progrès réalisés récemment dans le domaine des shampooings à base de polymères cationiques et de silicones, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

La présente invention vise à la satisfaction d'un tel besoin.

Les documents FR2795951 et FR2795953 décrivent des compositions cosmétiques comprenant un agent un tensioactif anionique, un tensioactif amphotère et un fructane cationique.

Ainsi, à la suite d'importantes recherches menées sur la question, il a maintenant été trouvé par la Demanderesse, de façon totalement inattendue et surprenante, qu'en utilisant une base lavante particulière, à savoir une base lavante associant au moins un tensioactif anionique et au moins un tensioactif choisi parmi les tensioactifs amphotères, non ioniques et cationiques dans un rapport particulier, comprenant en outre au moins un polysaccharide particulier, il est possible d'obtenir des compositions détergentes présentant d'excellentes propriétés cosmétiques, en particulier au niveau de la facilité de coiffage, du maintien, de la nervosité, du lissage et du démêlage des cheveux traités, ainsi qu'un très bon pouvoir lavant intrinsèque. Par ailleurs, les cheveux ne sont pas chargés, ils ont un toucher naturel et propre.

Toutes ces découvertes sont à la base de la présente invention.

Ainsi, selon la présente invention, il est maintenant proposé de nouvelles compositions notamment capillaires détergentes et conditionnantes comprenant, dans un milieu cosmétiquement acceptable, au moins un tensioactif anionique, au moins un tensioactif choisi parmi les tensioactifs amphotères, non ioniques et/ou cationiques et au moins un polysaccharide choisi parmi les hydrolysats d'amidon ayant un équivalent dextrose (DE) inférieur à 20 et les fructanes non ioniques ou anioniques, le rapport en poids tensioactif anionique / tensioactif amphotère, cationique et/ou non ionique étant supérieur ou égal à 1, les fructanes non ioniques ne contenant pas de groupes ioniques ou ionisables.
De préférence, ledit polysaccharide est soluble dans la composition. Encore plus préférentiellement, ledit polysaccharide est soluble dans l'eau.

Les hydrolysats d'amidon sont connus (voir Encyclopedia of Chemical Technology de Kirk-Othmer, 3e Ed., Vol.22, 1978, pp.499 à 521) et sont classés, selon leur apport en dextrose, en d'une part les sirops d'amidon et d'autre part les maltodextrines.
Les sirops d'amidon sont des hydrolysats d'amidon dont l'équivalent dextrose (DE) est supérieur à 20 et les maltodextrines sont des hydrolysats d'amidon dont le DE est inférieur à 20.
Le DE est le nombre de grammes de sucres réducteurs (considérés comme du dextrose) pour 100 g de matières sèches du produit. Le DE mesure donc l'intensité de l'hydrolyse de l'amidon puisque, plus le produit contient de petites molécules (telles que dextrose et maltose), plus son DE est élevé. Au contraire, plus le produit contient de grandes molécules (polysaccharides), plus son DE est bas.
Les sirops d'amidon (DE > 20) et les maltodextrines sont connus dans le domaine des soins capillaires comme étant utiles dans la préparation de compositions pour la fixation des cheveux.
Les sirops d'amidon (DE>20) utilisés à des concentrations supérieures à 10% en poids sont par ailleurs décrit pour améliorer la qualité de la mousse des compositions de shampooings.

L'invention a également pour objet l'utilisation en cosmétique des compositions ci-dessus pour le nettoyage et le conditionnement des cheveux.

Mais d'autres caractéristiques, aspects et avantages de l'invention apparaîtront encore plus clairement à la lecture de la description qui va suivre ainsi que des exemples concrets, mais nullement limitatifs, destinés à l'illustrer.

Dans le cadre de la présente demande, on entend par agent conditionneur tout agent ayant pour fonction l'amélioration des propriétés cosmétiques des cheveux, en particulier la douceur, le démêlage, le toucher, l'électricité statique.

Dans le cadre de la présente demande, on entend par fructane non ionique, un fructane qui ne contient pas de groupe ionique ou ionisable tels que notamment les amines primaires, secondaires, tertiaires ou les ammonium quaternaires.

Dans le cadre de la présente invention, on entend par polysaccharide soluble dans la composition ou dans l'eau, les polysaccharides de structure selon l'invention solubles dans l'eau ou la composition à une concentration supérieure ou égale à 0,1% en poids dans l'eau à environ 25°C, c'est à dire qu'ils forment dans ces conditions une solution macroscopiquement isotrope transparente.

Dans le cas où la composition contiendrait des agents autres que les polysaccharides, notamment des agents insolubles tels que des agents nacrants ou des agents conditionneurs insolubles, la détermination de la solubilité des polysaccharides dans la composition s'effectue en l'absence de ces agents insolubles.

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'aminés, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 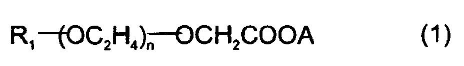 dans laquelle:
R₁ désigne un groupement alkyle ou alkylaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Parmi tous ces tensioactifs anioniques, on préfère utiliser plus particulièrement les sels d'alkylsulfates et d'alkyléthersulfates, ainsi que leurs mélanges.

### (ii) Tensioactif(s) amphotère(s) :

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂-N⁺(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHODIA CHIMIE.

### (iii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant 1 à 5 groupements glycérol et en particulier 1,5 à 4 ; les amines grasses polyéthoxylées ayant de préférence 2 à 30 moles d'oxyde d'éthylène; les esters d'acides gras du sorbitan oxyéthylénés ayant de 2 à 30 moles d'oxyde d'éthylène; les esters d'acides gras du sucrose, les esters d'acides gras de polyéthylèneglycol, les alkylpolyglycosides, les dérivés de N-alkyl glucamine, les oxydes d'aminés tels que les oxydes d'alkyl (C₁₀-C₁₄) aminés ou les oxydes de N-acylaminopropylmorpholine. On notera que les alkylpolyglycosides constituent des tensioactifs non-ioniques rentrant particulièrement bien dans le cadre de la présente invention.

### (iv) Tensioactifs cationiques :

Les tensioactifs cationiques peuvent être choisis parmi :
A) - les sels d'ammonium quaternaires de la formule générale (XII) suivante : 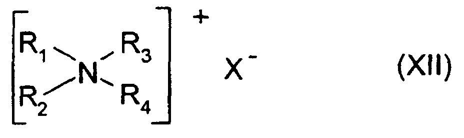 dans laquelle X⁻ est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate, et
   i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R4 désigne un radical alkyle, linéaire ou ramifié, comportant de 16 à 30 atomes de carbone.
      De préférence le tensioactif cationique est un sel (par exemple chlorure) de cétyl triméthyl ammonium.
   ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R3 et R4, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (XIII) suivante : 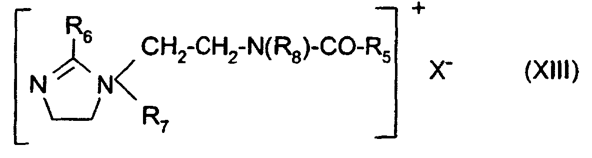 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (XIV) : 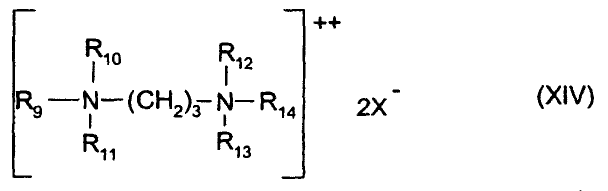 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (XV) suivante : 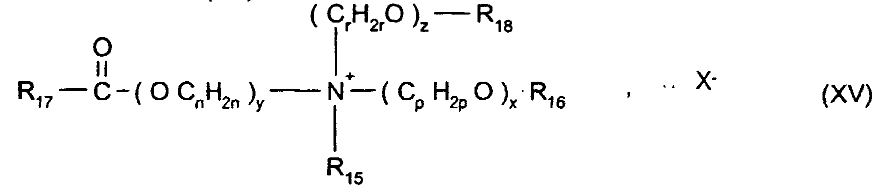 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
   - le radical 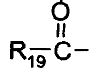
   - les radicaux R₂₀ hydrocarbonés en C₁-C₂₂ linéaires ou ramifiés, saturés ou insaturés,
   - l'atome d'hydrogène,
   - R₁₈ est choisi parmi:
   - le radical 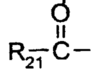
   - les radicaux R₂₂ hydrocarbonés en C₁-C₆ linéaires ou ramifiés, saturés ou insaturés,
   - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X- est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (XV) dans laquelle :
- R₁₅ désigne un radical méthyle ou éthyle,
- x et y sont égaux à 1 ;
- z est égal à 0 ou 1;
- n, p et r sont égaux à 2 ;
- R₁₆ est choisi parmi :
- le radical 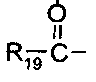
- les radicaux méthyle, éthyle ou hydrocarbonés en C₁₄-C₂₂
- l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
- le radical 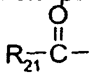
- l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

Parmi les sels d'ammonium quaternaire on préfère le chlorure de cétyltriméthylammonium, ou encore, le chlorure palmitamidopropyl triméthyl ammonium commercialisé sous la dénomination VARISOFT PA TC par la société GOLDSCHMIDT.

On utilise de préférence un agent tensioactif anionique choisi parmi les alkyl (C₁₂-C₁₄) sulfates de sodium, de triéthanolamine ou d'ammonium, les alkyl (C₁₂-C₁₄) éthersulfates de sodium, de triéthanolamine ou d'ammonium oxyéthylénés à 2,2 moles d'oxyde d'éthylène, parmi les alkyl(C₁₂-C₁₄) amido sulfates de sodium, de triéthanolamine ou d'ammonium, le cocoyl iséthionate de sodium et l'alphaoléfine(C₁₄-C₁₆) sulfonate de sodium en mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylamidobétaïnes et les alkylbétaïnes en particulier la cocobétaïne commercialisée sous la dénomination "DEHYTON AB 30" en solution aqueuse à 32 % de MA par la société COGNIS ou tel que les alkyl (C₈-C₂₀) amidoalkyl (C₁-C₆) bétaïnes en particulier la TEGOBETAINE® F 50 commercialisée par la société GOLDSCHMIDT.

A titre indicatif, les compositions détergentes conformes à l'invention présentent généralement les compositions suivantes :
(i) tensioactif(s) anionique(s) : de 2 à 50 % en poids, de préférence de 3 à 30 % en poids, par rapport au poids total de la composition détergente et plus particulièrement 3 à 20% en poids;
(ii) tensioactif(s) amphotère(s), non ionique(s) et/ou cationiques: de 1 à 50 % en poids, de préférence de 1 à 20 % en poids, par rapport au poids total de la composition et plus particulièrement 1 à 10% en poids.

La quantité totale des tensioactifs détergents décrits ci-dessus est généralement supérieure ou égale à 4 % en poids, de préférence comprise entre 5 % et 60 % en poids, mieux encore entre 5 % à 30 % en poids et au mieux entre 5 % et 20 % en poids par rapport au poids total de la composition.

Le rapport en poids tensioactif anionique / tensioactif amphotère, cationique et/ou non ionique est de préférence compris de 1 à 30, plus particulièrement de 2 à 20 et encore plus particulièrement de 3 à 10.

Selon un mode de réalisation particulier de l'invention, l'hydrolysat d'amidon a un DE allant de 1 à 18 et de préférence allant de 1 à 16, voire de 2 à 16.

Les maltodextrines utilisées selon l'invention peuvent être obtenues par l'hydrolyse partielle acide et/ou enzymatique de l'amidon. Divers procédés d'hydrolyse sont connus et ont été décrits de manière générale aux pages 511 et 512 de l'ouvrage Encyclopedia of Chemical Technology de Kirk-Othmer, 3e Ed., Vol.22, 1978.

L'amidon subissant cette hydrolyse peut provenir d'une origine variée mais de préférence du maïs, de fécule de pomme de terre, du tapioca, du riz ou du manioc. Les hydrolysats peuvent subir des modifications chimiques telles que par exemple une acétylation.

Les maltodextrines utilisables selon l'invention se présentent par exemple sous forme de poudre blanche ou d'une solution concentrée.

On citera, à titre d'exemple de maltodextrines, les produits vendus par la société Roquette sous les dénominations de Glucidex/ 1W (DE<3), 2 (DE<3), 6 (DE 5-8), 6B (DE 4-8), 9 (DE 8-10), 12 (DE 11-14), 17 (DE 15-18), et sous les dénominations de Glucidex/ IT6 (DE 5-8) et IT12 (DE 11-14), l'origine de ces maltodextrines étant l'amidon de maïs.

On peut également citer les produits vendus par la société National Starch sous les dénominations de N-Zorbit/ (DE<5) et Crystal Gum S/(DE<5), l'origine de ces maltodextrines étant l'amidon de manioc.

On peut également citer les produits vendus par la société Avebe sous les dénominations AVEBE MD 14 qui proviennent de fécule de pomme de terre.

Les fructanes ou fructosanes sont des oligosaccharides ou des polysaccharides comprenant un enchaînement d'unités anhydrofructose éventuellement associé à un plusieurs résidus saccharidiques différents du fructose. Les fructanes peuvent être linéaires ou ramifiés. Les fructanes peuvent être des produits obtenus directement à partir d'une source végétale ou microbienne ou bien des produits dont la longueur de chaîne a été modifiée (augmentée ou réduite) par fractionnement, synthèse ou hydrolyse en particulier enzymatique. Les fructanes ont généralement un degré de polymérisation de 2 à environ 1000 et de préférence de 3 à environ 60.
On distingue 3 groupes de fructanes. Le premier groupe correspond à des produits dont les unités fructose sont pour la plupart liées par des liaisons β-2-1.Ce sont des fructanes essentiellement linéaires tes que les inulines.
Le second groupe correspond également à des fructoses linéaires mais les unités fructose sont essentiellement liées par des liaisons β-2-6. Ces produits sont des levanes.
Le troisième groupe correspond à des fructanes mixtes, c'est à dire ayant des enchaînements β-2-6 et β-2-1. Ce sont des fructanes essentiellement ramifiés tes que les graminanes.

Les fructanes préférés sont les inulines et en particulier les inulines non ioniques. L'inuline peut être obtenue par exemple à partir de chicorée, de dahlia ou de topinambours.

La concentration en fructane ou en maltodextrine selon l'invention peut varier entre 0,01% et 5% en poids environ par rapport au poids total de la composition, et de préférence entre 0,1 et 5% environ et encore plus préférentiellement entre 0,2 et 3 % en poids.

Selon une variante préférée de l'invention, les compositions cosmétiques peuvent également contenir des agents de conditionnement des matières kératiniques.

L'invention a donc également pour objet de compositions cosmétiques comprenant en outre au moins un agent de conditionnement.

Ces compositions, lorsqu'elles sont appliquées sur les cheveux, possèdent de bonnes propriétés de conditionnement des cheveux, c'est-à-dire que les cheveux traités sont lisses, se démêlent facilement, sont doux au toucher. Les cheveux ont un aspect naturel et non chargé. De plus les cheveux ont plus de volume et se mettent facilement en forme.

Les compositions selon l'invention contenant des agents de conditionnement sont stables : en particulier, il ne se produit aucun relarguage des agents de conditionnement ou d'épaississement incontrôlé de la composition au cours du temps. Les compositions présentent enfin une texture non filante et fondante. La mousse se rince facilement.

Lorsque la composition contient au moins un agent de conditionnement, ils sont généralement choisis parmi les huiles de synthèse telles que les poly-α-oléfines, les huiles fluorées, les cires fluorées, les gommes fluorées, les esters d'acides carboxyliques, les polymères cationiques, les silicones, les huiles minérales, végétales ou animales, les céramides, les pseudocéramides et leurs mélanges.

Les polyoléfines sont de préférence des poly-α-oléfines et en particulier :
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence allant de 1000 à 15000.
   A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.
De tels produits sont vendus par exemple sous les dénominations ETHYLFLO par la société ETHYL CORP., et d'ARLAMOL PAO par la société ICI.

Les huiles minérales pouvant être utilisées dans les compositions de l'invention sont choisies préférentiellement dans le groupe formé par les hydrocarbures, tels que l'hexadécane et l'huile de paraffine ;

Selon un mode de réalisation préféré, la composition comprend en outre au moins un polymère cationique.

De manière encore plus générale, au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 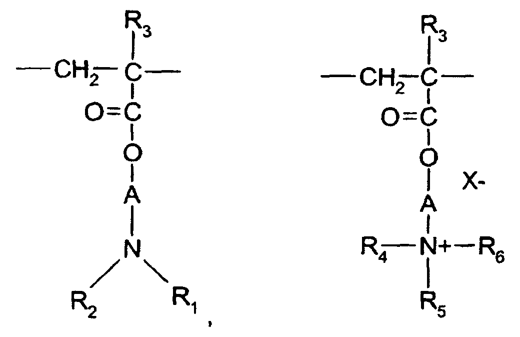 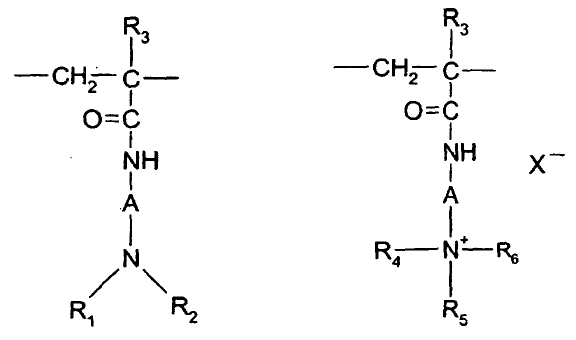 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle; X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthylaminopropyle quaternisé tel que le produit vendu sous la dénomination "GAFQUAT HS 100" par la société ISP.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "CELQUAT® L 200" et "CELQUAT® H 100" par la Société National Starch.
(4) Les polysaccharides cationiques décrits plus particulièrement dans les brevets US 3 589 578 et 4 031 307 tels que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR® C13 S, JAGUAR® C 15, JAGUAR® C 17 ou JAGUAR® C162 par la société RHODIA CHIMIE.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions aminés tertiaires, quaternisées. De tels polymères sont notamment décrits dans les brevets français 2.252.840 et 2.368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) les polymères obtenus par réaction d'une polyalkylène polyamine comportant deux groupements aminé primaire et au moins un groupement aminé secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) les cyclopolymères d'alkyl diallyl amine ou de dialkyl diallyl ammonium tels que les homopolymères ou copolymères comportant comme constituant principal de la chaîne des motifs répondant aux formules (Va) ou (Vb) : 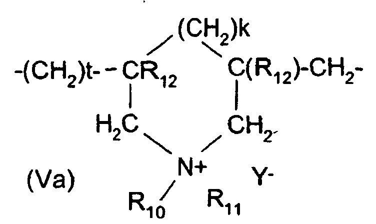 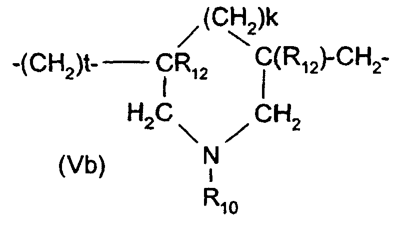 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "Merquat 100" par la société NALCO (et ses homologues de faibles masses moléculaires moyenne en poids) et les copolymères de chlorure de diallyldiméthylammonium et d'acrylamide commercialisés sous la dénomination "MERQUAT 550".
(10) le polymère de diammonium quaternaire contenant des motifs récurrents répondant à la formule (VI) : 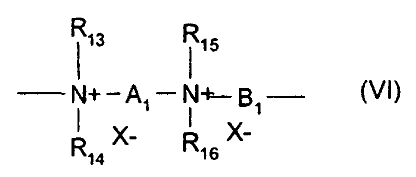 formule (VI) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ; n varie de 1 à 6

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 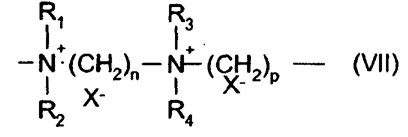 dans laquelle R₁, R₂, R₃ et R₄ identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (VII) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(11) les polymères de polyammonium quaternaires constitués de motifs de formule (VIII): 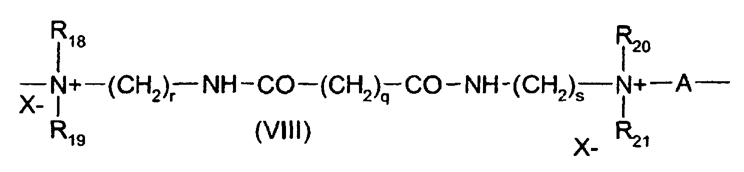 formule dans laquelle:
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogènure,
   A désigne un radical d'un dihalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(12) Les polymères quaternaires de vinylpyrrolidone et de vinylimidazole tels que par exemple les produits commercialisés sous les dénominations Luviquat® FC 905, FC 550 et FC 370 par la société B.A.S.F.
(13) Les polyamines comme le Polyquart® H vendu par COGNIS, référencé sous le nom de « POLYETHYLENEGLYCOL (15) TALLOW POLYAMINE » dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société AMERCHOL, les cyclopolymères cationiques, en particulier les homopolymères ou copolymères de chlorure de diméthyldiallylammonium, vendus sous les dénominations « MERQUAT 100», « MERQUAT 550 » et « MERQUAT S » par la société NALCO, les polysaccharides cationiques tels que les gommes de guar modifiées par un sel de 2,3-époxypropyl triméthylammonium, les polymères quaternaires de vinylpyrrolidone et de vinylimidazole et leurs mélanges.

Selon l'invention, le ou les polymères cationiques peuvent représenter de 0,001 % à 10 % en poids, de préférence de 0,005 % à 5% en poids et plus particulièrement de 0,01 à 3% en poids par rapport au poids total de la composition finale.

Les silicones utilisables conformément à l'invention sont en particulier des polyorganosiloxanes insolubles dans la composition et peuvent se présenter sous forme d'huiles, de cires, de résines ou de gommes.

La viscosité des silicones est par exemple mesurée à 25°C selon la norme ASTM 445 Appendice C.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition allant de 60° C à 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique : 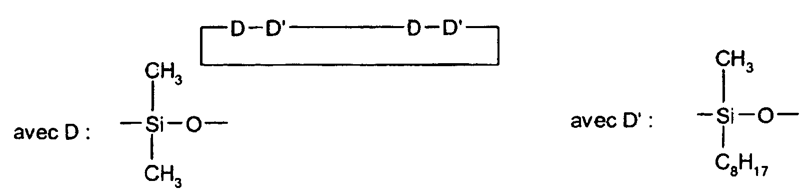
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s.

Parmi ces polyalkylsiloxanes, on peut citer à titre non limitatif les produits commerciaux suivants :
- les huiles de la série MIRASIL commercialisées par la société RHODIA CHIMIE, telles que par exemple l'huile MIRASIL DM 500 000 ;
- les huiles de la série 200 de la société DOW CORNING telles que plus particulièrement la DC200 de viscosité 60 000 cSt;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE .

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles MIRASIL DPDM de RHODIA CHIMIE ;
. les huiles des séries RHODORSIL 70 633 et 763 de RHODIA CHIMIE ;
. l'huile DOW CORNING 556 COSMETIC GRAD FLUID de DOW CORNING;
. les silicones de la série PK de BAYER comme le produit PK20 ;
. les silicones des séries PN, PH de BAYER comme les produits PN1000 et PH1000;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334.
- des groupements acyloxyalkyle tels que par exemple les polyorganosiloxanes décrits dans,le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 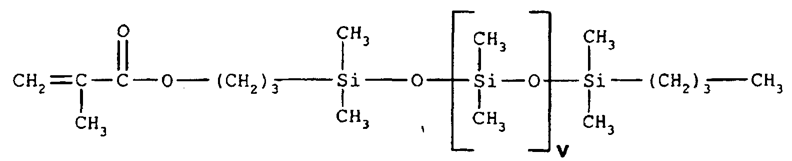 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise allant de 0,2 à 2,5 m²/s à 25° C telles que les huiles de la séries DC200 de DOW CORNING en particulier celle de viscosité 60 000 cSt, des séries MIRASIL DM et plus particulièrement l'huile MIRASIL DM 500 000 commercialisées par la société RHODIA CHIMIE ou l'huile de silicone AK 300.000 de la société WACKER, les polyalkylsiloxanes à groupements terminaux diméthylsilanol tels que les diméthiconol ou les polyalkylarylsiloxanes tels que l'huile MIRASIL DPDM commercialisée par la société RHODIA CHIMIE ;
- les polysiloxanes à groupements aminés tels que les amodiméthicones ou les triméthylsilylamodiméthicones ;

Selon la présente invention, les composés de type céramide sont notamment les céramides et/ou les glycocéramides et/ou les pseudocéramides et/ou les néocéramides, naturels ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique;
- le N-docosanoyl N-méthyl-D-glucamine
ou les mélanges de ces composés.

Les esters utilisables selon l'invention sont notamment choisis parmi les esters d'acides mono- di-, tri-carboxyliques aliphatiques saturés ou insaturés, linéaires ou ramifiés en C₁₀-C₃₀ et d'alcools ou de polyols aliphatiques saturés ou insaturés, linéaires ou ramifiés en C₁-C₈ , les esters d'acides mono- di-, tri-carboxyliques aliphatiques saturés ou insaturés, linéaires ou ramifiés en C₂-C₈ et d'alcools ou de polyols aliphatiques saturés ou insaturés, linéaires ou ramifiés en C₁₀-C₃₀,

Parmi les esters selon l'invention, on peut citer le lactate de cétyle ; le lactate d'alkyle en C₁₂-C₁₅ ; le lactate d'isostéaryle ; le lactate de lauryle ; le lactate de linoléyle ; le lactate d'oléyle ; les palmitates d'éthyle et d'isopropyle, les myristates d'alkyles en C1-C5 tels que le myristate d'isopropyle, de butyle, le stéarate d'isobutyle ; le malate de dioctyle, le laurate d'hexyle, le laurate de 2-hexyldécyle.

De préférence, l'agent de conditionnement insoluble est choisi parmi les polymères cationiques, les silicones et leurs mélanges.

Les agents de conditionnement insolubles peuvent être dispersés dans les compositions sous forme de particules ayant généralement une taille moyenne en volume (mesurée par l'une des techniques décrites dans l'article Particle size Analysis, Anal. Chem. 1995, 67, 257-272) comprise entre 2 nanomètres et 100 microns, de préférence de 10 nm à 50 microns.

Selon l'invention, les agents conditionneurs peuvent représenter de 0,001 % à 10 % en poids, de préférence de 0,005 % à 5 % en poids, et encore plus préférentiellement de 0,01 % à 3 % en poids, du poids total de la composition finale.

Le milieu cosmétiquement acceptable est de préférence constitué par de l'eau ou un mélange d'eau et de solvants cosmétiquement ou dermatologiquement acceptables tels que des monoalcools, des polyalcools, des éthers de glycol qui peuvent être utilisés seuls ou en mélange. L'eau représente de préférence de 30 à 98% en poids et de préférence de 50 à 98% en poids par rapport au poids total de la composition.

On peut citer plus particulièrement les monoalcools tels que l'éthanol, l'iso-propanol, les polyalcools tels que le diéthylèneglycol, la glycérine, les éthers de glycol, les alkyléthers de glycol ou de diéthylèneglycol.

Les compositions détergentes selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'iso-propanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants (associatifs ou non associatifs). On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir de préférence jusqu'à 5 % d'agents nacrants ou opacifiants bien connus dans l'état de la technique tels que par exemple les alcools gras supérieurs à C16, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement contenir en outre au moins un additif choisi parmi les synergistes de mousses tels que des 1,2-alcanediols en C₁₀-C₁₈ ou des alcanolamides gras dérivés de mono ou de diéthanolamine d'acide gras en C8-C24, les filtres solaires siliconés ou non, les polymères anioniques ou non ioniques, les polymères amphotères, les protéines, les hydrolysats de protéines, les hydroxyacides, les vitamines, les provitamines tels que le panthénol, et tout autre additif classiquement utilisé dans le domaine cosmétique qui n'affecte pas les propriétés des compositions selon l'invention.

Selon une variantes préférée de l'invention, les compositions contiennent en outre une vitamine, une provitamine et/ou un hydroxyacide tel que par exemple l'acide citrique ou l'acide tartrique.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Ces additifs sont présents dans la composition selon l'invention dans des proportions pouvant aller de 0,00001 à 20% en poids par rapport au poids total de la composition. La quantité précise de chaque additif est déterminée facilement par l'homme du métier selon sa nature et sa fonction.

Ces compositions peuvent se présenter sous la forme de liquides, de crèmes ou de gels et elles conviennent principalement au lavage, au soin des matières kératiniques en particulier des cheveux et de la peau et encore plus particulièrement des cheveux.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, cette application étant suivie d'un rinçage à l'eau.

Les compositions conformes à l'invention sont également utilisables comme gels douche pour le lavage et le conditionnement des cheveux et/ou de la peau, auquel cas ils sont appliqués sur la peau et/ou les cheveux humides et sont rincés après application.

Les compositions de l'invention peuvent encore se présenter sous la forme de compositions lavantes pour la peau, et en particulier sous la forme de solutions ou de gels pour le bain ou la douche ou de produits démaquillants.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Des exemples concrets, mais nullement limitatif, illustrant l'invention vont maintenant être donnés.

### EXEMPLE 1

On a réalisé un shampooing A conforme à l'invention et un shampooing B non conforme à l'invention de compositions suivantes :

| Eng | A | B |
|---|---|---|
| Lauryl éther sulfate de sodium (2.2 OE) en solution aqueuse à 70% de MA | 15,4 gMA | 15,4 gMA |
| Cocoyl bétaine en solution aqueuse à 30% de MA | 2,4 gMA | 2,4 gMA |
| Inuline de racine de chicorée (fructose/glucose) en poudre (FIBRULINE de COSUCRA ) | 0,5 g | - |
| Poly dimethylsiloxane (visco: 500.000 cSt) (MIRASIL DM 500.000 de RHODIA) | 1.5 g | 1.5 g |
| Hydroxyéthyl Cellulose quaternisée par du Chlorure de 2,3 époxypropyl triméthyl Ammonium (JR400 de AMERCHOL) | - | 0.4 |
| Mélange 1-(hexadécyloxy)-2octadécanol / Alcool cétylique | 2.5 g | 2.5 g |
| Monoisopropanolamide d'acides de coprah | 1g | 1 g |
| Acide polyacrylique réticulé Carbopol 980 de NOVEON | 0.2 g | 0.2 g |
| Parfum, conservateur | qs | qs |
| N-oléoyl dihydrosphingosine | 0.01 g | 0.01 g |
| pH = 6.5+/-0.5 | | |
| Eau qsp | 100 g | 100 g |

On effectue un shampooing en appliquant environ 12 g de la composition sur des cheveux préalablement mouillés. On fait mousser le shampooing puis on rince abondamment à l'eau.

La composition a une texture épaisse et très fondante lors de l'application sur des cheveux humides. Sa rinçabilité est bonne.

Un panel d'experts a trouvé que les compositions conformes à l'invention confèrent aux cheveux, après rinçage, un remarquable effet traitant qui se manifeste notamment par une facilité de démêlage, de coiffage, ainsi qu'un lissage et une douceur remarquables des cheveux.

Les cheveux lavés avec la composition A ont plus de volume et se mettent plus facilement en forme que les cheveux traités avec la composition B. Ils ont un toucher moins chargés que ceux traités avec la composition B.

### EXEMPLE 2

On a réalisé un shampooing conforme à l'invention de composition suivante :

| En g | A | B |
|---|---|---|
| Lauryl éther sulfate de sodium (2.2 OE) en solution aqueuse à 70% de MA | 11 ,2 gMA | 11 ,2 gMA |
| Mélange cocoyl amidopropyl bétaine / mono-laurate de glycéryle en solution aqueuse à 30 % de MA ( TEGOBETAINE HS de GOLDSCHMIDT) | 1,92 gMA | 1,92 gMA |
| Inuline de racine de chicorée (fructose/glucose) en poudre (FIBRULINE de COSUCRA ) | 0,6 g | - |
| Acide citrique | 3g | 3g |
| Hydroxyéthyl Cellulose quaternisée par du Chlorure de 2,3 époxypropyl triméthyl Ammonium (JR400 de AMERCHOL) | - | 0.3 |
| Acide tartrique | 0,2 g | 0,2 g |
| Extrait hydroglycolique de feuille de thé vert (HERBASOL extrait thé vert de COSMETOCHEM) | 0.01 g | 0.01 g |
| Extrait hydroglycolique de pamplemousse jaune (VEGETOL HYDROGLYCOLIQUE PAMPLEMOUSSE GR 403 de GATTEFFOSSE) | 0.01 g | 0.01 g |
| Parfum, conservateur | qs | qs |
| N-oléoyl dihydrosphingosine | 0.01 g | 0.01 g |
| Ammoniaque à 20,5% de NH3 qs pH | 5,3 | 5,3 |
| Eau qsp | 100 g | 100 g |

Les cheveux lavés avec la composition A se démêlent facilement , sont lisses. Ils ont plus de volume et se mettent plus facilement en forme que les cheveux traités avec la composition B.
Ils ont un toucher moins chargés que ceux traités avec la composition B.

### EXEMPLE 3

On a réalisé un shampooing de composition suivante :

| En g | A | B |
|---|---|---|
| Lauryl éther sulfate de sodium (2.2 OE) en solution aqueuse à 70% de MA | 15,4gMA | 15,4 gMA |
| Cocoyl bétaine en solution aqueuse à 30% de MA | 2,4 gMA | 2,4 gMA |
| Maltodextrine de pomme de terre (MD14 de AVEBE) | 1.5 g | - |
| Poly diméthylsiloxane (visco : 500.000 cSt) (MIRASIL DM 500.000 de RHODIA) | 1.5 g | 1.5 g |
| Hydroxyéthyl Cellulose quaternisée par du Chlorure de 2,3 époxypropyl triméthyl Ammonium (JR400 de AMERCHOL) | - | 0.4 |
| Mélange 1-(hexadécyloxy)-2octadécanol / Alcool cétylique | 2.5 g | 2.5 g |
| Monoisopropanolamide d'acides de coprah | 1g | 1g |
| Acide polyacrylique réticulé Carbopol 980 de NOVEON | 0.2 g | 0.2 g |
| Parfum, conservateur | qs | qs |
| N-oléoyl dihydrosphingosine | 0.01 g | 0.01 g |
| pH = 6.5 +/- 0.5 | | |
| Eau qsp | 100 g | 100 g |

Les cheveux lavés avec la composition A se démêlent facilement , sont lisses. Ils ont plus de volume et se mettent plus facilement en forme que les cheveux traités avec la composition B. Ils ont un toucher moins chargés que ceux traités avec la composition B.

## Revendications

1. Composition détergente et conditionnante, **caractérisée par le fait qu'**elle comprend, dans un milieu cosmétiquement acceptable, au moins un tensioactif anionique, au moins un tensioactif amphotère, cationique et/ou non ionique, au moins un polysaccharide choisi parmi les fructanes non ioniques ou anioniques et les hydrolysat d'amidon ayant un équivalent dextrose (DE) inférieur à 20,
et le rapport en poids tensioactif anionique / tensioactif amphotère, cationique et/ou non ionique étant supérieur ou égal à 1,
les fructanes non ioniques ne contenant pas de groupes ioniques ou ionisables.

2. Composition selon la revendication 1, **caractérisée par** te fait que le ou lesdits tensioactifs anioniques sont présents à raison de 2 à 50 % en poids, de préférence de 3 à 30 % en poids, par rapport au poids total de la composition.

3. Composition selon l'une quelconque des revendications précédentes, **caractérisée par le fait que** le ou lesdits tensioactifs amphotères, non ioniques et/ou cationiques sont présents à raison de 1 à 50 % en poids, de préférence de 1 à 20 % en poids, par rapport au poids total de la composition.

4. Composition selon l'une quelconque des revendications précédentes, **caractérisée par le fait que** le rapport en poids tensioactif anionique/ tensioactifs amphotères, non ioniques et/ou cationiques va de 1 à 30, plus particulièrement de 2 à 20 et encore plus particulièrement de 3 à 10.

5. Composition selon l'une quelconque des revendications précédentes, **caractérisée par le fait que** ledit tensioactif anionique est choisi les alkyl(C₁₂-C₁₄) sulfates de sodium, de triéthanolamine ou d'ammonium, les alkyl (C₁₂-C₁₄)éthersulfates de sodium, de triéthanoiamine ou d'ammonium oxyéthylénés à 2,2 moles d'oxyde d'éthylène, parmi les alkyl(C₁₂-C₁₄)amido sulfates de sodium, de triéthanolamine ou d'ammonium, le cocoyl iséthionate de sodium et l'alphaoléfine(C₁₄-C₁₆) sulfonate de sodium.

6. Composition selon rune quelconque des revendications précédentes, **caractérisée par le fait que** ledit tensioactif amphotère est choisi parmi les dérivés d'amines secondaires ou tertiaires aliphatiques, dans lesquels le radical aliphatique est une chaîne linéaire ou ramifiée comportant 8 à 22 atomes de carbone et contenant au moins un groupe anionique, les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

7. Composition selon l'une quelconque des revendications précédentes, **caractérisée par le fait que** les polysaccharides sont solubles dans la composition.

8. Composition selon l'une quelconque des revendications précédentes, **caractérisée par le fait que** les polysaccharides sont solubles dans l'eau.

9. Composition selon l'une quelconque des revendications précédentes, **caractérisée par le fait que** les hydrolysats d'amidon ont un DE allant de 1 à 18, et de préférence allant de 1 à 16.

10. Composition selon l'une quelconque des revendications 1 à 8, **caractérisée par le fait que** le polysaccharide est l'inuline.

11. Composition selon la revendication 10, **caractérisée par le fait que** l'inuline est non ionique.

12. Composition selon l'une quelconque des revendications précédentes, **caractérisée par le fait que** les polysaccharides sont présents en une proportion comprise entre 0,01 et 5 %, et de préférence entre 0,1 et 5 % en poids et encore plus particulièrement entre 0,2 et 3% en poids par rapport au poids total de la composition.

13. Composition selon l'une quelconque des revendications précédentes, **caractérisée en ce qu'**elle comprend en outre au moins un agent de conditionnement.

14. Composition selon la revendication précédente, **caractérisée en ce que** l'agent de conditionnement est choisi parmi les huiles fluorées, les cires fluorées, les gommes fluorées, les esters d'acides carboxyliques, les silicones, les huiles de synthèse, les polymères cationiques, les huiles minérales, végétales ou animales, les cires végétales, les céramides, les pseudocéramides, et leurs mélanges.

15. Composition selon la revendication 14, **caractérisée en ce que** l'agent de conditionnement est choisi parmi les polymères cationiques, les silicones et leurs mélanges.

16. Composition selon l'une des revendications 14 à 15, **caractérisée par le fait que** les silicones sont choisies parmi les polyalkylsiloxanes, les polyarylsiloxanes, les polyalkylarylsiloxanes, les gommes et les résines de silicones, les polyorganosiloxanes modifiés par des groupements organofonctionnels ainsi que leurs mélanges.

17. Composition selon la revendication 16, **caractérisée par le fait que** :
(a) les polyalkylsiloxanes sont choisis parmi :
- les polydiméthylsiloxanes à groupements terminaux triméthylsilyle ;
- les polydiméthylsiloxanes à groupements terminaux diméthylsilanol ;
- les polyalkyl(C₁-C₂₀)siloxanes ;
(b) les polyalkylarylsiloxanes sont choisis parmi :
- les polydiméthylméthylphénylsiloxanes, les polydiméthyldiphényl siloxanes linéaires et/ou ramifiés de viscosité comprise entre 1.10⁻⁵ et 5.10⁻²m²/s à 25°C ;
(c) les gommes de silicone sont choisies parmi les polydiorganosiloxanes ayant des masses moléculaires moyennes en nombre comprises entre 200 000 et 1 000 000 utilisés seuls ou sous forme de mélange dans un solvant ;
(d) les résines sont choisies parmi les résines constituées d'unités : R₃ Si O_{1/2}, R₂ Si O_{2/2}, R Si O_{3/2}, Si O_{4/2}
dans lesquelles R représente un groupement hydrocarboné ayant de 1 à 16 atomes de carbone ou un groupement phényle ;

18. Composition selon la revendication 16, **caractérisée par le fait que** les groupements organofonctionnels sont choisis parmi :
a) des groupements aminés substitués ou non
b) des groupements (per)fluorés,
c) des groupements thiols ;
d) des groupements carboxylates,
e) des groupements hydroxylés,
f) des groupements alcoxylés
g) des groupements acyloxyalkyls
h) des groupements amphotères;
i) des groupements bisulfites.
j) des groupements hydroxyacylamino,
k) des groupements acide carboxylique
l) des groupements sulfoniques
m) des groupements sulfates ou thiosulfates;

19. Composition selon l'une quelconque des revendications 14 à 18, **caractérisée par le fait que** les silicones sont choisies parmi les polydiméthylsiloxanes linéaires à groupements terminaux triméthylsilyle, les polydiméthylsiloxanes linéaires à groupements terminaux hydroxydiméthylsilyle, les résines de silicone, les amodiméthicones ou les triméthylsilylamodiméthicones.

20. Composition selon la revendication 14, **caractérisée par le fait que** les huiles de synthèse sont des polyoléfines de type polybutène, hydrogéné ou non, ou de type polydécène, hydrogéné ou non.

21. Composition selon la revendication 14, **caractérisée par le fait que** les huiles animales ou végétales sont choisies dans le groupe formé par les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone en particulier alkyle ou alkényle, les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote.

22. Composition selon la revendication 14, **caractérisée par le fait que** les cires végétales sont choisies parmi la cire de Carnauba, la cire de Candelilla, l'ozokérite, la cire d'olivier, la cire de riz, la cire de jojoba hydrogénée ou les cires absolues de fleurs telles que la cire essentielle de fleur de cassis, les cires marines.

23. Composition selon la revendication 14, **caractérisée par le fait que** les céramides et pseudocéramides répondent à la formule générale (I): 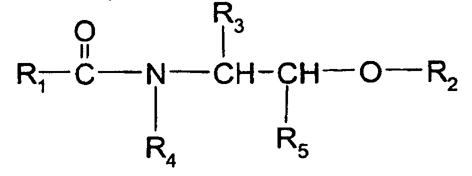 dans laquelle :
- R₁ désigne :
- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C_{35,} le ou les hydroxyles du radical R₇ pouvant être estérifié par un acide gras saturé ou insaturé, linéaire ou ramifié, éventuellement mono ou polyhydroxylé, en C₁-C₃₅;
- soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent.
- soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)_{m,} sulfogalactosyle, phosphoryléthylamine ou phosphoryléthylammonium, R₃ pouvant également être substitué par un ou plusieurs radicaux alkyle en C₁-C₁₄ ;
de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

24. Composition selon la revendication 14, **caractérisée en ce que** le céramide est choisi dans le groupe constitué par :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol,
- le 2-N-palmitoylamino-hexadécane-1,3-diol,
- le bis-(N-hydroxyéthyl N-cétyl) malonamide,
- le N-(2-hydroxyéthyl)-N-(3-cétyloxy-2-hydroxypropyl) amide d'acide cétylique)
- le N-docosanoyl N-méthyl-D-glucamine ou les mélanges de ces composés.

25. Composition selon l'une quelconque des revendications 13 à 24, **caractérisée par le fait que** le ou les agents de conditionnement sont présents dans des concentrations allant de 0,0001 à 20% en poids par rapport au poids total de la composition et de préférence de 0,001 à 10 % en poids et plus préférentiellement entre 0,005 et 3% en poids.

26. Composition selon l'une quelconque des revendications 1 à 25, **caractérisée par le fait qu'**elle comprend en outre au moins un polymère cationique.

27. Composition selon la revendication 26, **caractérisée par le fait que** ledit polymère cationique est choisi parmi les dérivés d'éther de cellulose quaternaires, les cyclopolymères cationiques, les polysaccharides cationiques, les polymères quaternaires de vinylpyrrolidone et de vinylimidazole et leurs mélanges.

28. Composition selon la revendication 27, **caractérisée par le fait que** ledit cyclopolymère est choisi parmi les homopolymères du chlorure de diallyldiméthylammonium et les copolymères du chlorure de diallyldiméthylammonium et d'acrylamide.

29. Composition selon la revendication 27, **caractérisée par le fait que** lesdits dérivés d'éther de cellulose quaternaires sont choisis parmi les hydroxyéthyl-celluloses ayant réagi avec un époxyde substitué par un groupement triméthylammonium.

30. Composition selon la revendication 27, **caractérisée par le fait que** lesdits polysaccharides cationiques sont choisis parmi les gommes de guar modifiées par un sel de 2,3-époxypropyl triméthylammonium.

31. Composition selon l'une quelconque des revendications 26 à 30 **caractérisée en ce que** le polymère cationique représente de 0,001 % à 10 % en poids, de préférence de 0,005 % à 5 % en poids, et encore plus préférentiellement de 0,01 % à 3 % en poids, du poids total de la composition.

32. Composition selon l'une quelconque des revendications 1 à 31, **caractérisée par le fait qu'**elle comprend en outre au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les polymères anioniques ou non ioniques ou amphotères, les protéines, les hydrolysats de protéines, les hydroxyacides, les vitamines, les provitamines tels que le panthénol, et tout autre additif classiquement utilisé dans le domaine cosmétique.

33. Composition selon l'une quelconque des revendications 1 à 31, **caractérisée par le fait qu'**elle comprend en outre au moins une vitamine, une provitamine et/ou un hydroxyacide.

34. Composition selon l'une quelconque des revendications 1 à 33, **caractérisée par le fait que** la quantité totale de tensioactifs est comprise dans une concentration allant de 4 % et 60 % en poids, mieux encore de 5 % à 30 % en poids par rapport au poids total de la composition.

35. Composition selon l'une quelconque des revendications précédentes, **caractérisée par le fait qu'**elle se présente sous forme de shampooing, de composition pour la permanente, le défrisage, la coloration ou la décoloration des cheveux, de composition à rincer à appliquer entre les deux étapes d'une permanente ou d'un défrisage, de compositions lavantes pour la peau.

36. Utilisation d'une composition telle définie dans l'une quelconque des revendications 1 à 32 comme shampooing des matières kératiniques.

37. Procédé de lavage et de conditionnement des fibres kératiniques telles que les cheveux consistant à appliquer sur lesdites fibres mouillées une quantité efficace d'une composition telle que définie à l'une quelconque des revendications précédentes, puis à effectuer un rinçage à l'eau après un éventuel temps de pause.

## Patentansprüche

1. Reinigende und konditionierende Zusammensetzung, **dadurch gekennzeichnet, dass** sie in einem kosmetisch akzeptablen Medium mindestens einen anionischen grenzflächenaktiven Stoff, mindestens einen amphoteren, kationischen und/oder nichtionischen grenzflächenaktiven Stoff und mindestens ein Polysaccharid enthält, das unter den nichtionischen oder anionischen Fructanen und den Stärkehydrolysaten mit einem Dextroseäquivalent (DE) unter 20 ausgewählt ist,
und das Gewichtsverhältnis anionischer grenzflächenaktiver Stoff/amphoterer, kationischer und / oder nichtionischer grenzflächenaktiver Stoff 1 beträgt oder darüber liegt,
wobei die nichtionischen Fructane keine ionischen oder ionisierbaren Gruppen aufweisen.

2. Zusammensetzung nach Anspruch 1, **dadurch gekennzeichnet, dass** der oder die anionische(n) grenzflächenaktive(n) Stoff(e) in einer Menge von 2 bis 50 Gew.-% und vorzugsweise 3 bis 30 Gew.-%, bezogen auf das Gesamtgewicht der Zusammensetzung, vorliegen.

3. Zusammensetzung nach einem der vorhergehenden Ansprüche, **dadurch gekennzeichnet, dass** der oder die amphotere(n), nichtionische(n) und/oder kationische(n) grenzflächenaktive(n) Stoff(e) in einer Menge von 1 bis 50 Gew.-% und vorzugsweise 1 bis 20 Gew.-%, bezogen auf das Gesamtgewicht der Zusammensetzung, vorliegen.

4. Zusammensetzung nach einem der vorhergehenden Ansprüceh, **dadurch gekennzeichnet, dass** das Gewichtsverhältnis anionischer grenzflächenaktiver Stoff/amphoterer, nichtionischer und/oder kationischer grenzflächenaktiver Stoff im Bereich von 1 bis 30, insbesondere 2 bis 20 und besonders 3 bis 10 liegt.

5. Zusammensetzung nach einem der vorhergehenden Ansprüche, **dadurch gekennzeichnet, dass** der anionische grenzflächenaktive Stoff unter den Natriumalkyl(C₁₂₋₁₄)sulfaten, Triethanolaminalkyl(C₁₂₋₁₄)sulfaten oder Ammoniumalkyl(C₁₂₋₁₄)sulfaten, Natriumalkyl(C₁₂₋₁₄)ethersulfaten, Triethanolaminalkyl(C₁₂₋₁₄)ethersulfaten oder Ammoniumalkyl(C₁₂₋₁₄-)ethersulfaten, die mit 2,2 mol Ethylenoxid ethoxyliert sind, Natriumalkyl(C₁₂₋₁₄)amidosulfaten, Triethanolaminalkyl(C₁₂₋₁₄)amidosulfaten oder Ammoniumalkyl(C₁₂₋₁₄)amidosulfaten, Natriumcocoylisethionat und Natrium-α-olefin(C₁₄₋₁₆)sulfonat ausgewählt ist.

6. Zusammensetzung nach einem der vorhergehenden Ansprüche, **dadurch gekennzeichnet, dass** der amphotere grenzflächenaktive Stoff unter den aliphatischen, sekundären oder tertiären Aminderivaten, worin die aliphatische Gruppe eine geradkettige oder verzweigte Gruppe mit 8 bis 22 Kohlenstoffatomen bedeutet und mindestens eine anionische Gruppe aufweist, Alkyl(C₈₋₂₀)betainen, Sulfobetainen, Alkyl(C₈₋₂₀)amidoalkyl(C₁₋₆)betainen oder Alkyl(C₈₋₂₀)amidoalkyl(C₁₋₆)sulfobetainen ausgewählt ist.

7. Zusammensetzung nach einem der vorhergehenden Ansprüche, **dadurch gekennzeichnet, dass** die Polysaccharide in der Zusammensetzung löslich sind.

8. Zusammensetzung nach einem der vorhergehenden Ansprüche, **dadurch gekennzeichnet, dass** die Polysaccharide wasserlöslich sind.

9. Zusammensetzung nach einem der vorhergehenden Ansprüche, **dadurch gekennzeichnet, dass** die Stärkehydrolysate einen DE-Wert von 1 bis 18 und vorzugsweise 1 bis 16 aufweisen.

10. Zusammensetzung nach einem der Ansprüche 1 bis 8, **dadurch gekennzeichnet, dass** es sich bei dem Polysaccharid um Inulin handelt.

11. Zusammensetzung nach Anspruch 10, **dadurch gekennzeichnet, dass** das Inulin nichtionisch ist.

12. Zusammensetzung nach einem der vorhergehenden Ansprüche, **dadurch gekennzeichnet, dass** die Polysaccharide in einem Mengenanteil von 0,01 bis 5 Gew.-%, vorzugsweise 0,1 bis 5 Gew.-% und insbesondere 0,2 bis 3 Gew.-%, bezogen auf das Gesamtgewicht der Zusammensetzung, enthalten sind.

13. Zusammensetzung nach einem der vorhergehenden Ansprüche, **dadurch gekennzeichnet, dass** sie ferner mindestens ein Konditioniermittel enthält.

14. Zusammensetzung nach dem vorhergehenden Anspruch, **dadurch gekennzeichnet, dass** das Konditioniermittel unter den fluorierten Ölen, fluorierten Wachsen, fluorierten Gummis, Carbonsäureestern, Siliconen, synthetischen Ölen, kationischen Polymeren, Mineralölen, pflanzlichen Ölen oder tierischen Ölen, pflanzlichen Wachsen, Ceramiden, Pseudoceramiden und deren Gemischen ausgewählt ist.

15. Zusammensetzung nach Anspruch 14, **dadurch gekennzeichnet, dass** das Konditioniermittel unter den kationischen Polymeren, Siliconen und deren Gemischen ausgewählt ist.

16. Zusammensetzung nach einem der Ansprüche 14 bis 15, **dadurch gekennzeichnet, dass** die Silicone unter den Polyalkylsiloxanen, Polyarylsiloxanen, Polyalkylarylsiloxanen, Silicongummis und Siliconwachsen, mit organofunktionellen Gruppen modifizierten Polyorganosiloxanen sowie deren Gemischen ausgewählt sind.

17. Zusammensetzung nach Anspruch 16, **dadurch gekennzeichnet, dass**:
(a) die Polyalkylsiloxane ausgewählt sind unter:
- Polydimethylsiloxanen mit endständigen Trimethylsilylgruppen;
- Polydimethylsiloxanen mit endständigen Dimethylsilanolgruppen;
- Polyalkyl(C₁₋₂₀)siloxanen;
(b) die Polyalkylarylsiloxane ausgewählt sind unter:
- Polydimethylmethylphenylsiloxanen, Polydimethyldiphenylsiloxanen, die geradkettig und/oder verzweigt vorliegen und bei 25 °C eine Viskosität von 1·10⁻⁵ bis 5.10⁻² m²/s aufweisen;
(c) die Silicongummis unter den Polydiorganosiloxanen mit einer zahlenmittleren Molmasse von 200 000 bis 1 000 000 ausgewählt sind, die einzeln oder in Form eines Gemisches in einem Lösungsmittel verwendet werden; und
(d) die Harze unter den Harzen ausgewählt sind, die aus den folgenden Einheiten bestehen: R₃ Si O_{1/2}, R₂ Si O_{2/2}, R Si O_{3/2}, Si O_{4/2}, worin R eine Kohlenwasserstoffgruppe mit 1 bis 16 Kohlenstoffatomen oder eine Phenylgruppe bedeutet.

18. Zusammensetzung nach Anspruch 16, **dadurch gekennzeichnet, dass** die organofunktionellen Gruppen ausgewählt sind unter:
a) substituierten oder unsubstituierten aminierten Gruppen,
b) (per)fluorierten Gruppen,
c) Thiolgruppen,
d) Carboxylatgruppen,
e) hydroxylierten Gruppen,
f) alkoxylierten Gruppen,
g) Acyloxyalkylgruppen,
h) amphoteren Gruppen,
i) Bisulfitgruppen,
j) Hydroxyacylaminogruppen,
k) Carboxygruppen,
l) Sulfonsäuregruppen und
m) Sulfat- oder Thiosulfatgruppen.

19. Zusammensetzung nach einem der Ansprüche 14 bis 18, **dadurch gekennzeichnet, dass** die Silicone unter den geradkettigen Polydimethylsiloxanen mit endständigen Trimethylsilylgruppen, geradkettigen Polydimethylsiloxanen mit endständigen Hydroxydimethysilylgruppen, Siliconharzen, Amodimethiconen oder Trimethylsilylamodimethiconen ausgewählt sind.

20. Zusammensetzung nach Anspruch 14, **dadurch gekennzeichnet, dass** die synthetischen Öle Polyolefine vom Typ der hydrierten oder nicht hydrierten Polybutene oder vom Typ der hydrierten oder nicht hydrierten Polydecene sind.

21. Zusammensetzung nach Anspruch 14, **dadurch gekennzeichnet, dass** die tierischen oder pflanzlichen Öle unter Sonnenblumenöl, Maisöl, Sojaöl, Avocadoöl, Jojobaöl, Kürbiskernöl, Traubenkernöl, Sesamöl, Haselnussöl, Fischölen, Glyceryltricaprocaprylat oder den pflanzlichen oder tierischen Ölen der Formel R₉COOR₁₀, worin R₉ den Rest einer höheren Fettsäure mit 7 bis 29 Kohlenstoffatomen und Rio eine geradkettige oder verzweigte Kohlenwasserstoffgruppe mit 3 bis 30 Kohlenstoffatomen, insbesondere Alkyl oder Alkenyl, bedeutet, und natürlichen oder synthetischen etherischen Ölen wie beispielsweise Eucalyptusöl, Lavandinöl, Lavendelöl, Vetiveröl, Öl aus Litsea cubeba, Citronenöl, Sandelholzöl, Rosmarinöl, Kamilleöl, Muskatnussöl, Zimtöl, Ysopöl, Kümmelöl, Orangenöl, Geraniolöl, Wacholderöl und Bergamotteöl ausgewählt sind.

22. Zusammensetzung nach Anspruch 14, **dadurch gekennzeichnet, dass** die pflanzlichen Wachse unter Carnaubawachs, Candelillawachs, Ozokerit, Olivenwachs, Reiswachs, hydriertem Jojobawachs oder den reinen Blütenwachsen, wie etherischem Wachs aus Cassisblüten und den marinen Wachsen ausgewählt ist.

23. Zusammensetzung nach Anspruch 14, **dadurch gekennzeichnet, dass** die Ceramide und Pseudoceramide der folgenden allgemeinen Formel (I) entsprechen: 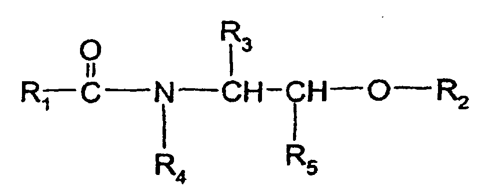 wobei in der Formel:
- R₁ bedeutet:
- entweder eine geradkettige oder verzweigte, gesättigte oder ungesättigte Kohlenwasserstoffgruppe mit 1 bis 50 und vorzugsweise 5 bis 50 Kohlenstoffatomen, wobei diese Gruppe mit einer oder mehreren Hydroxygruppen substituiert sein kann, die gegebenenfalls mit einer Säure R₇COOH verestert sind, wobei R₇ eine gesättigte oder ungesättigte, geradkettige oder verzweigte, gegebenenfalls mono- oder polyhydroxylierte Kohlenwasserstoffgruppe mit 1 bis 35 Kohlenstoffatomen ist und wobei die Hydroxygruppe(n) der Gruppe R₇ mit einer gesättigten oder ungesättigten, geradkettigen oder verzweigten, gegebenenfalls mono- oder polyhydroxylierten Fettsäure mit 1 bis 35 Kohlenstoffatomen verestert sein kann (können);
- oder eine Gruppe R"-(NR-CO)-R', wobei R ein Wasserstoffatom oder eine mono- oder polyhydroxylierte Kohlenwasserstoffgruppe mit 1 bis 20 Kohlenstoffatomen bedeutet, wobei die Gruppe vorzugsweise monohydroxyliert ist, und R' und R" Kohlenwasserstoffgruppen sind, wobei die Summe der Kohlenstoffatome dieser Gruppen im Bereich von 9 bis 30 liegt und wobei R' eine zweiwertige Gruppe ist;
- oder eine Gruppe R₈-O-CO-(CH₂)ₚ, wobei R₈ eine C₁₋₂₀-Kohlenwasserstoffgruppe ist und p eine ganze Zahl im Bereich von 1 bis 12 bedeutet;
- R₂ unter einem Wasserstoffatom, einer Gruppe vom Saccharidtyp, insbesondere einer Gruppe (Glycosyl)ₙ, (Galactosyl)ₘ oder Sulfogalactosyl, einem Sulfat- oder Phosphatrest, einer Phosphorylethylamingruppe oder einer Phosphorylethylammoniumgruppe ausgewählt ist, wobei n eine ganze Zahl von 1 bis 4 und m eine ganze Zahl von 1 bis 8 bedeutet;
- R₃ ein Wasserstoffatom oder eine gesättigte oder ungesättigte, gegebenenfalls hydroxylierte C₁₋₃₃-Kohlenwasserstoffgruppe bedeutet, wobei die Hydroxygruppe(n) mit einer anorganischen Säure oder einer Säure R₇COOH verestert sein kann (können),
wobei R₇ die oben angegebenen Bedeutungen aufweist, und die Hydroxygruppe(n) mit einer Gruppe (Glycosyl)ₙ, (Galactosyl)ₘ, Sulfogalactosyl, Phorphorylethylamin oder Phosphorylethylammonium verethert sein kann (können), wobei R₃ auch mit einer oder mehreren C₁₋₁₄-Alkylgruppen substituiert sein kann; wobei R₃ vorzugsweise eine α-Hydroxyalkylgruppe mit 15 bis 26 Kohlenstoffatomen bedeutet, wobei die Hydroxygruppe gegebenenfalls mit einer α-Hydroxysäure mit 16 bis 30 Kohlenstoffatomen verestert ist;
- R₄ ein Wasserstoffatom, Methyl, Ethyl, eine gesättigte oder ungesättigte, geradkettige oder verzweigte Kohlenwasserstoffgruppe mit 3 bis 50 Kohlenstoffatomen, die gegebenenfalls hydroxyliert ist, eine Gruppe -CH₂-CHOH-CH₂-O-R₆, wobei R₆ eine C₁₀₋₂₆-Kohlenwasserstoffgruppe ist, oder eine Gruppe R₈-O-CO-(CH₂)ₚ bedeutet, wobei R₈ eine C₁₋₂₀-Kohlenwasserstoffgruppe ist und p eine ganze Zahl von 1 bis 12 bedeutet;
- R₅ ein Wasserstoffatom oder eine gesättigte oder ungesättigte, geradkettige oder verzweigte, gegebenenfalls mono- oder polyhydroxylierte C₁₋₃₀-Kohlenwasserstoffgruppe ist, wobei die Hydroxygruppe(n) mit einer Gruppe (Glycosyl)ₙ, (Galactosyl)ₘ, Sulfogalactosyl, Phosphorylethylamin oder Phosphorylethylammonium verethert sein kann (können),
mit der Maßgabe, dass R₄ kein Wasserstoffatom, keine Methylgruppe und keine Ethylgruppe bedeutet, wenn R₃ und R₅ Wasserstoff bedeuten oder wenn R₃ Wasserstoff und R₅ Methyl bedeutet.

24. Zusammensetzung nach Anspruch 14, **dadurch gekennzeichnet, dass** das Ceramid ausgewählt ist unter:
- 2-N-Linoleoylamino-octadecan-1,3-diol,
- 2-N-Oleoylamino-octadecan-1,3-diol,
- 2-N-Palmitoylamino-octadecan-1,3-diol,
- 2-N-Stearoylamino-octadecan-1,3-diol,
- 2-N-Behenoylamino-octadecan-1,3-diol,
- 2-N-[2-Hydroxy-palmitoyl]-amino-octadecan-1,3-diol,
- 2-N-Stearoylamino-octadecan-1,3,4-triol;
- 2-N-Palmitoylamino-hexadecan-1,3-diol,
- Bis(N-hydroxyethyl-N-cetyl)-malonamid,
- N-(2-Hydroxyethyl)-N-(3-cetyloxy-2-hydroxypropyl)-amid von Cetylsäure,
- N-Docosanoyl-N-methyl-D-glucamin oder den Gemischen dieser Verbindungen.

25. Zusammensetzung nach einem der Ansprüche 13 bis 24, **dadurch gekennzeichnet, dass** das oder die Konditioniermittel in einer Konzentration von 0,0001 bis 20 Gew.-%, bezogen auf das Gesamtgewicht der Zusammensetzung, vorzugsweise 0,001 bis 10 Gew.-% und besonders bevorzugt 0,005 bis 3 Gew.-% enthalten sind.

26. Zusammensetzung nach einem der Ansprüche 1 bis 25, **dadurch gekennzeichnet, dass** sie ferner mindestens ein kationisches Polymer enthält.

27. Zusammensetzung nach Anspruch 26, **dadurch gekennzeichnet, dass** das kationische Polymer unter den quaternisierten Celluloseetherderivaten, kationischen Cyclopolymeren, kationischen Polysacchariden, quartären Polymeren von Vinylpyrrolidon und Vinylimidazol und deren Gemischen ausgewählt ist.

28. Zusammensetzung nach Anspruch 27, **dadurch gekennzeichnet, dass** das Cyclopolymer unter den Homopolymeren von Diallyldimethylammoniumchlorid und den Copolymeren von Diallyldimethylammoniumchlorid und Acrylamid ausgewählt ist.

29. Zusammensetzung nach Anspruch 27, **dadurch gekennzeichnet, dass** die quartären Celluloseetherderivate unter den Hydroxyethylcellulosen ausgewählt sind, die mit einem mit einer Trimethylammoniumgruppe substituierten Epoxid umgesetzt wurden.

30. Zusammensetzung nach Anspruch 27, **dadurch gekennzeichnet, dass** die kationischen Polysaccharide unter den mit einem 2,3-Epoxypropyltrimethylammoniumsalz modifizierten Guargummen ausgewählt sind.

31. Zusammensetzung nach einem der Ansprüche 26 bis 30, **dadurch gekennzeichnet, dass** das kationische Polymer 0,001 bis 10 Gew.-%, vorzugsweise 0,005 bis 5 Gew.-% und noch bevorzugter 0,01 bis 3 Gew.-% des Gesamtgewichts der Zusammensetzung ausmacht.

32. Zusammensetzung nach einem der Ansprüche 1 bis 31, **dadurch gekennzeichnet, dass** sie ferner mindestens einen Zusatzstoff enthält, der unter den Schaumsynergisten, wie 1,2-Alkandiolen mit 10 bis 18 Kohlenstoffatomen oder Alkanolamiden von Fettsäuren, die von Mono- oder Diethanolamin abgeleitet sind, siliconhaltigen oder nicht siliconhaltigen Sonnenschutzfiltern, anionischen oder nichtionischen oder amphoteren Polymeren, Proteinen, Proteinhydrolysaten, Hydroxysäuren, Vitaminen, Provitaminen, wie Panthenol, oder beliebigen anderen Zusatzstoffen ausgewählt ist, die gewöhnlich in der Kosmetik verwendet werden.

33. Zusammensetzung nach einem der Ansprüche 1 bis 31, **dadurch gekennzeichnet, dass** sie ferner mindestens ein Vitamin, ein Provitamin und/ oder eine Hydroxysäure enthält.

34. Zusammensetzung nach einem der Ansprüche 1 bis 33, **dadurch gekennzeichnet, dass** die Gesamtmenge der grenzflächenaktiven Stoffe im Bereich einer Konzentration von 4 bis 60 Gew.-% und besser 5 bis 30 Gew.-%, bezogen auf das Gesamtgewicht der Zusammensetzung, liegt.

35. Zusammensetzung nach einem der vorhergehenden Ansprüche, **dadurch gekennzeichnet, dass** sie als Haarwaschmittel, Zusammensetzung für permanente Verformungen, Entkräuselungen, Färbungen oder Entfärbungen der Haare, Zusammensetzung, die ausgespült wird und zwischen den beiden Schritten einer permanenten Verformung oder Entkräuselung aufgetragen wird, oder als reinigende Zusammensetzung für die Haut vorliegt.

36. Verwendung einer Zusammensetzung nach einem der Ansprüche 1 bis 32 als Shampoo für Keratinsubstanzen.

37. Verfahren zur Reinigung und Konditionierung von Keratinfasern, beispielsweise der Haare, das darin besteht, auf die feuchten Fasern eine wirksame Menge einer Zusammensetzung nach einem der vorhergehenden Ansprüche aufzubringen und anschließend nach einer gegebenenfalls abgewarteten Einwirkzeit mit Wasser zu spülen.

## Claims

1. Detergent and conditioning composition, **characterized in that** it comprises, in a cosmetically acceptable medium, at least one anionic surfactant, at least one amphoteric, cationic and/or nonionic surfactant and at least one polysaccharide chosen from nonionic or anionic fructans and starch hydrolysates with a dextrose equivalent (DE) of less than 20,
and the anionic surfactant / amphoteric, cationic and/or nonionic surfactant weight ratio being greater than or equal to 1, the nonionic fructan not containing ionic or ionizable groups.

2. Composition according to Claim 1, **characterized in that** said anionic surfactant(s) is (are) present in a proportion of from 2% to 50% by weight and preferably from 3% to 30% by weight relative to the total weight of the composition.

3. Composition according to either of the preceding claims, **characterized in that** said amphoteric, nonionic and/or cationic surfactant(s) is (are) present in a proportion of from 1% to 50% by weight and preferably from 1% to 20% by weight relative to the total weight of the composition.

4. Composition according to any one of the preceding claims, **characterized in that** the anionic surfactant / amphoteric, nonionic and/or cationic surfactants weight ratio ranges from 1 to 30, more particularly from 2 to 20 and even more particularly from 3 to 10.

5. Composition according to any one of the preceding claims, **characterized in that** said anionic surfactant is chosen from sodium, triethanolamine or ammonium (C₁₂-C₁₄)alkyl sulfates, sodium, triethanolamine or ammonium (C₁₂-C₁₄)alkyl ether sulfates oxyethylenated with 2.2 mol of ethylene oxide, from sodium, triethanolamine or ammonium (C₁₂-C₁₄)alkylamido sulfates, sodium cocoyl isethionate and sodium (C₁₄-C₁₆)-α-olefin sulfonate.

6. Composition according to any one of the preceding claims, **characterized in that** said amphoteric surfactant is chosen from aliphatic secondary or tertiary amine derivatives, in which the aliphatic radical is a linear or branched chain containing 8 to 22 carbon atoms and containing at least one anionic group, (C₈-C₂₀)alkylbetaines, sulfobetaines, (C₈-C₂₀)alkylamido (C₁-C₆)alkylbetaines or (C₈-C₂₀)alkylamido (C₁-C₆)alkylsulfobetaines.

7. Composition according to any one of the preceding claims, **characterized in that** the polysaccharides are soluble in the composition.

8. Composition according to any one of the preceding claims, **characterized in that** the polysaccharides are water-soluble.

9. Composition according to any one of the preceding claims, **characterized in that** the starch hydrolysates have a DE ranging from 1 to 18 and preferably ranging from 1 to 16.

10. Composition according to any one of Claims 1 to 8, **characterized in that** the polysaccharide is inulin.

11. Composition according to Claim 10, **characterized in that** the inulin is nonionic.

12. Composition according to any one of the preceding claims, **characterized in that** the polysaccharides are present in a proportion of between 0.01% and 5%, preferably between 0.1% and 5% by weight and even more particularly between 0.2% and 3% by weight, relative to the total weight of the composition.

13. Composition according to any one of the preceding claims, **characterized in that** it also comprises at least one conditioner.

14. Composition according to the preceding claim, **characterized in that** the conditioner is chosen from fluoro oils, fluoro waxes, fluoro gums, carboxylic acid esters, silicones, synthetic oils, cationic polymers, mineral, plant or animal oils, plant waxes, ceramides and pseudoceramides, and mixtures thereof.

15. Composition according to Claim 14, **characterized in that** the conditioner is chosen from cationic polymers and silicones, and mixtures thereof.

16. Composition according to either of Claims 14 and 15, **characterized in that** the silicones are chosen from polyalkylsiloxanes, polyarylsiloxanes, polyalkylarylsiloxanes, silicone gums and resins, and polyorganosiloxanes modified with organofunctional groups, and also mixtures thereof.

17. Composition according to Claim 16, **characterized in that**:
(a) the polyalkylsiloxanes are chosen from:
- polydimethylsiloxanes containing trimethylsilyl end groups;
- polydimethylsiloxanes containing dimethylsilanol end groups;
- poly(C₁-C₂₀)alkylsiloxanes;
(b) the polyalkylarylsiloxanes are chosen from:
- linear and/or branched polydimethylmethylphenylsiloxanes and polydimethyldiphenylsiloxanes with a viscosity of between 1x10⁻⁵ and 5x10⁻² m²/s at 25°C;
(c) the silicone gums are chosen from polydiorganosiloxanes with number-average molecular masses of between 200 000 and 1 000 000, which are used alone or in the form of a mixture in a solvent;
(d) the resins are chosen from resins consisting of units: R₃ Si O_{1/2}, R₂ Si O_{2/2}, R Si O_{3/2}, Si O_{4/2}
in which R represents a hydrocarbon-based group containing from 1 to 16 carbon atoms or a phenyl group.

18. Composition according to Claim 16, **characterized in that** the organofunctional groups are chosen from:
a) substituted or unsubstituted amine groups,
b) (per)fluoro groups,
c) thiol groups,
d) carboxylate groups,
e) hydroxylated groups,
f) alkoxylated groups,
g) acyloxyalkyl groups,
h) amphoteric groups,
i) bisulfite groups,
j) hydroxyacylamino groups,
k) carboxylic acid groups,
l) sulfonic groups,
m) sulfate or thiosulfate groups.

19. Composition according to any one of Claims 14 to 18, **characterized in that** the silicones are chosen from linear polydimethylsiloxanes containing trimethylsilyl end groups, linear polydimethylsiloxanes containing hydroxydimethylsilyl end groups, silicone resins, amodimethicones and trimethylsilylamodimethicones.

20. Composition according to Claim 14, **characterized in that** the synthetic oils are polyolefins of hydrogenated or nonhydrogenated polybutene type or of hydrogenated or nonhydrogenated polydecene type.

21. Composition according to Claim 14, **characterized in that** the animal or plant oils are chosen from the group formed by sunflower oil, maize oil, soybean oil, avocado oil, jojoba oil, marrow oil, grapeseed oil, sesame oil, hazelnut oil, fish oils, glyceryl tricaprocaprylate, or plant or animal oils of formula R₉COOR₁₀ in which R₉ represents a higher fatty acid residue containing from 7 to 29 carbon atoms and R₁₀ represents a linear or branched hydrocarbon-based chain containing from 3 to 30 carbon atoms, in particular alkyl or alkenyl, natural or synthetic essential oils such as, for example, eucalyptus oil, lavandin oil, lavender oil, vetiver oil, litsea cubeba oil, lemon oil, sandalwood oil, rosemary oil, camomile oil, savory oil, nutmeg oil, cinnamon oil, hyssop oil, caraway oil, orange oil, geraniol oil, cade oil and bergamot oil.

22. Composition according to Claim 14, **characterized in that** the plant waxes are chosen from carnauba wax, candelilla wax, ozokerite, olive wax, rice wax, hydrogenated jojoba wax or the absolute waxes of flowers such as the essential wax of blackcurrant flower, and marine waxes.

23. Composition according to Claim 14, **characterized in that** the ceramides and pseudoceramides correspond to the general formula (I): 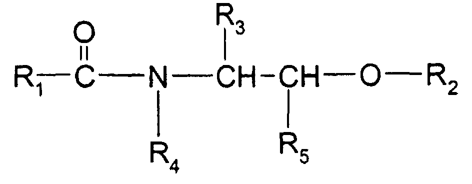 in which:
- R₁ denotes:
- either a saturated or unsaturated, linear or branched C₁-C₅₀, preferably C₅-C₅₀, hydrocarbon-based radical, it being possible for this radical to be substituted with one or more hydroxyl groups which may be esterified with an acid R₇COOH, R₇ being a saturated or unsaturated, linear or branched, optionally mono- or polyhydroxylated C₁-C₃₅ hydrocarbon-based radical, it being possible for the hydroxyl(s) of the radical R₇ to be esterified with a saturated or unsaturated, linear or branched, optionally mono- or polyhydroxylated C₁-C₃₅ fatty acid;
- or a radical R"-(NR-CO)-R', R denotes a hydrogen atom or a mono- or polyhydroxylated, preferably monohydroxylated, C₁-C₂₀ hydrocarbon-based radical, R' and R" are hydrocarbon-based radicals, the sum of the carbon atoms of which is between 9 and 30, R' being a divalent radical;
- or a radical R₈-O-CO-(CH₂)ₚ, R₈ denotes a C₁-C₂₀ hydrocarbon-based radical, p is an integer ranging from 1 to 12.
- R₂ is chosen from a hydrogen atom, a radical of saccharide type, in particular a (glycosyl)ₙ, (galactosyl)ₘ or sulfogalactosyl radical, a sulfate or phosphate residue, a phosphorylethylamine radical and a phosphorylethylammonium radical, in which n is an integer ranging from 1 to 4 and m is an integer ranging from 1 to 8;
- R₃ denotes a hydrogen atom or a saturated or unsaturated, hydroxylated or nonhydroxylated C₁-C₃₃ hydrocarbon-based radical, it being possible for the hydroxyl(s) to be esterified with an inorganic acid or an acid R₇COOH, R₇ having the same meanings as above, it being possible for the hydroxyl(s) to be etherified with a (glycosyl)ₙ, (galactosyl)ₘ, sulfogalactosyl, phosphorylethylamine or phosphorylethylammonium radical, it being also possible for R₃ to be substituted with one or more C₁-C₁₄ alkyl radicals; preferably, R₃ denotes a C₁₅-C₂₆ α-hydroxyalkyl radical, the hydroxyl group optionally being esterified with a C₁₆-C₃₀ α-hydroxy acid;
- R₄ denotes a hydrogen atom, a methyl or ethyl radical, a saturated or unsaturated, linear or branched, optionally hydroxylated C₃-C₅₀ hydrocarbon-based radical or a radical -CH₂-CHOH-CH₂-O-R₆ in which R₆ denotes a C₁₀-C₂₆ hydrocarbon-based radical or a radical R₈-O-CO-(CH₂)ₚ, R₈ denotes a C₁-C₂₀ hydrocarbon-based radical, p is an integer ranging from 1 to 12;
- R₅ denotes a hydrogen atom or a saturated or unsaturated, linear or branched, optionally mono- or polyhydroxylated C₁-C₃₀ hydrocarbon-based radical, it being possible for the hydroxyl(s) to be etherified with a (glycosyl)ₙ, (galactosyl)ₘ, sulfogalactosyl, phosphorylethylamine or phosphorylethylammonium radical,
with the proviso that when R₃ and R₅ denote hydrogen or when R₃ denotes hydrogen and R₅ denotes methyl, then R₄ does not denote a hydrogen atom or a methyl or ethyl radical.

24. Composition according to Claim 14, **characterized in that** the ceramide is chosen from the group consisting of:
- 2-N-linoleoylaminooctadecane-1,3-diol,
- 2-N-oleoylaminooctadecane-1,3-diol,
- 2-N-palmitoylaminooctadecane-1,3-diol,
- 2-N-stearoylaminooctadecane-1,3-diol,
- 2-N-behenoylaminooctadecane-1,3-diol,
- 2-N-[2-hydroxypalmitoyl]aminooctadecane-1,3-diol,
- 2-N-stearoylaminooctadecane-1,3,4-triol,
- 2-N-palmitoylaminohexadecane-1,3-diol,
- bis(N-hydroxyethyl-N-cetyl)malonamide,
- cetylic acid N-(2-hydroxyethyl)-N-(3-cetyloxy-2-hydroxypropyl)amide,
- N-docosanoyl-N-methyl-D-glucamine, or mixtures of these compounds.

25. Composition according to any one of Claims 13 to 24, **characterized in that** the conditioner(s) is (are) present in concentrations ranging from 0.0001% to 20% by weight, preferably from 0.001% to 10% by weight and more preferably between 0.005% and 3% by weight, relative to the total weight of the composition.

26. Composition according to any one of Claims 1 to 25, **characterized in that** it also comprises at least one cationic polymer.

27. Composition according to Claim 26, **characterized in that** said cationic polymer is chosen from quaternary cellulose ether derivatives, cationic cyclopolymers, cationic polysaccharides and quaternary polymers of vinylpyrrolidone and of vinylimidazole, and mixtures thereof.

28. Composition according to Claim 27, **characterized in that** said cyclopolymer is chosen from diallyldimethylammonium chloride homopolymers and copolymers of diallyldimethylammonium chloride and of acrylamide.

29. Composition according to Claim 27, **characterized in that** said quaternary cellulose ether derivatives are chosen from hydroxyethylcelluloses that have reacted with an epoxide substituted with a trimethylammonium group.

30. Composition according to Claim 27, **characterized in that** said cationic polysaccharides are chosen from guar gums modified with a 2,3-epoxypropyltrimethylammonium salt.

31. Composition according to any one of Claims 26 to 30, **characterized in that** the cationic polymer represents from 0.001% to 10% by weight, preferably from 0.005% to 5% by weight and even more preferably from 0.01% to 3% by weight, of the total weight of the composition.

32. Composition according to any one of Claims 1 to 31, **characterized in that** it also comprises at least one additive chosen from foam synergists such as C₁₀-C₁₈ 1,2-alkanediols or fatty alkanolamides derived from monoethanolamine or from diethanolamine, silicone or nonsilicone sunscreens, anionic or nonionic or amphoteric polymers, proteins, protein hydrolysates, hydroxy acids, vitamins, provitamins such as panthenol, and any other additive conventionally used in cosmetics.

33. Composition according to any one of Claims 1 to 31, **characterized in that** it also comprises at least one vitamin, one provitamin and/or one hydroxy acid.

34. Composition according to any one of Claims 1 to 33, **characterized in that** the total amount of surfactants is within a concentration ranging from 4% to 60% by weight and better still from 5% to 30% by weight relative to the total weight of the composition.

35. Composition according to any one of the preceding claims, **characterized in that** it is in the form of a shampoo, a composition for permanent-waving, relaxing, dyeing or bleaching the hair, a rinse-out composition to be applied between the two steps of a permanent-waving or relaxing operation, or a washing composition for the skin.

36. Use of a composition as defined in any one of Claims 1 to 32 as a shampoo for keratin materials.

37. Process for washing and conditioning keratin fibres such as the hair, which consists in applying to said wet fibres an effective amount of a composition as defined in any one of the preceding claims, followed by rinsing with water after an optional action time.
